# EUROPEAN PATENT APPLICATION

(11) **EP 0 683 395 A2**
(43) Date of publication of application: **22.11.1995**
(21) Application number: 95107507.6
(22) Date of filing: 17.05.1995
(51) Int. Cl.: G01N 33/483, G01N 15/06, G01N 21/01, G01N 21/86, G01N 33/04, C12Q 1/04

(54) **Method and apparatus for analyzing heterogeneous liquids, particularly for counting somatic cells in milk**

(30) Priority: 18.05.1994 IT MI941006
(71) Applicant: PLURISTANDARD dott. Guido Motolese S.r.l., I-20123 Milano (IT)
(72) Inventor: Motolese, Guido, I-20123 Milan (IT)
(74) Representative: Modiano, Guido, Dr.-Ing.

(57) **Abstract**

The present invention relates to a method and an apparatus for analyzing heterogeneous liquids, particularly for counting somatic cells in vaccine milk. The process consists in taking a sample of the liquid to be analyzed; in evenly spreading a film of the liquid of the sample onto a ribbon-like element that can move along a preset path; and in analyzing the film of liquid placed on the ribbon-like element by means of a microscope that is arranged along the path of the ribbon-like element downstream of the station for spreading the liquid onto the ribbon-like element along the direction of motion of the ribbon-like element.

## Description

The present invention relates to a method and an apparatus for analyzing heterogeneous liquids, particularly for counting somatic cells in vaccine milk.

It is known that one of the parameters that are constantly monitored in all the most advanced countries by companies distributing or processing cow milk is the number of somatic cells contained per unit volume. This number is subjected to specific legal limits, since an excess of somatic cells, especially if originating from blood (leukocytes, macrophages, lymphocytes), entails undesirable changes to the chemical and physical structure of the milk.

The interest of dairy farmers in controlling the cell content of raw milk, however, is dictated not only and not so much by the financial penalty to which they are subjected by the purchasing companies according to the number of cells per milliliter, even if it is below the allowed limits, but also and most of all by the fact that an abnormal increase in somatic cells in the milk of a cow is an indication of the presence of an infection of the udder which, if not promptly treated, temporarily or permanently reduces the productivity of the cow. The onset of clinical mastitis in a dairy animal forces the farmer to treat it with antibiotics and therefore to lose its production throughout the course of the therapy until the antibiotic content of the milk returns below the values set by the law. The farmer who notices symptoms of the disorder in a cow usually takes a sample of milk and sends it to the nearest analysis laboratory. The direct cost of the analysis is low, but the indirect costs, such as for example the costs arising from the time required, transport, and others, are high.

Accordingly, the need to have analyzers that can be part of the current stable equipment is increasingly felt and desired in veterinary circles as part of the actions aimed at improving quality in agroindustrial activities.

Currently, in order to check the number of somatic cells in vaccine milk, apart from the classical method of direct counting under the microscope, which can be performed only by specialized operators, with a considerable expenditure of time, there are two quick methods that yield results that are acknowledged as acceptable by the authorities in charge: an impedance-measurement method and a cytofluorometric method.

The impedance-measurement method essentially consists in passing the sample to be measured through a narrow channel (approximately 100 µm in diameter) on two sides whereof the electrodes of the cell of an impedance meter are arranged so as to face each other. The device counts one pulse for each variation in impedance between the two electrodes caused by the transit of a cell. Only one model of this type of instrument validated within the EEC is currently available.

The cytofluorometric method instead consists in preliminarily marking the cells contained in the sample with a fluorescent substance and then in making the sample flow through a forced passage, on which an optical system for energizing the marker substance bonded to each individual cell is focused. The fluorescent response signal is collected by the same optical system or by a separate one, depending on the geometry of the apparatus, is converted into an electric signal by an appropriate sensor, and is then sent to an electronic processing and counting unit.

One of the basic problems that hinders the adoption of these instruments directly in the stable is their high cost, which depends mainly on the need to prevent previous samples from contaminating the sample being measured. This fact makes indispensable to perform accurate flushing of all the parts that make contact with the sample for any reason (pumps, pipes, measurement point).

The aim of the present invention is to solve the problem described above by providing a method for analyzing heterogeneous liquids, particularly for counting somatic cells in cow milk, which can be performed by an apparatus that is very sturdy and simple to use, so that it can also be used in a stable.

Within the scope of this aim, an object of the invention is to provide an apparatus that has a modest cost with respect to currently commercially available devices for counting somatic cells in cow milk.

Another object of the invention is to provide an apparatus that offers the greatest assurances against the possibility of contaminations of the milk samples being analyzed.

This aim, these objects, and others which will become apparent hereinafter are achieved by a method for analyzing heterogeneous liquids, particularly for counting somatic cells in vaccine milk, characterized in that it consists in taking a sample of the liquid to be analyzed; in evenly spreading a film of the sample liquid on a ribbon-like element that can move along a preset path; in analyzing the film of liquid placed on said ribbon-like element by means of a microscope arranged along said path downstream of the station for spreading said liquid onto the ribbon-like element along the direction of motion of said ribbon-like element.

In order to carry out the method according to the invention, an apparatus is preferably used, characterized in that it comprises: means for taking a sample of the liquid to be analyzed; a ribbon-like element that runs along a preset path; means for moving said ribbon-like element along said path; means for delivering a film of the liquid of said sample onto one face of said ribbon-like element, said delivery means facing said ribbon-like element; and means for the optical analysis of the film of the sample liquid spread on said ribbon-like element, said means being arranged downstream of said delivery means along the direction of motion of said ribbon-like element along said path.

Further characteristics and advantages of the invention will become apparent from the description of a preferred but not exclusive embodiment of the method and of the apparatus for carrying it out, according to the invention, illustrated only by way of non-limitative example in the accompanying drawings, wherein:

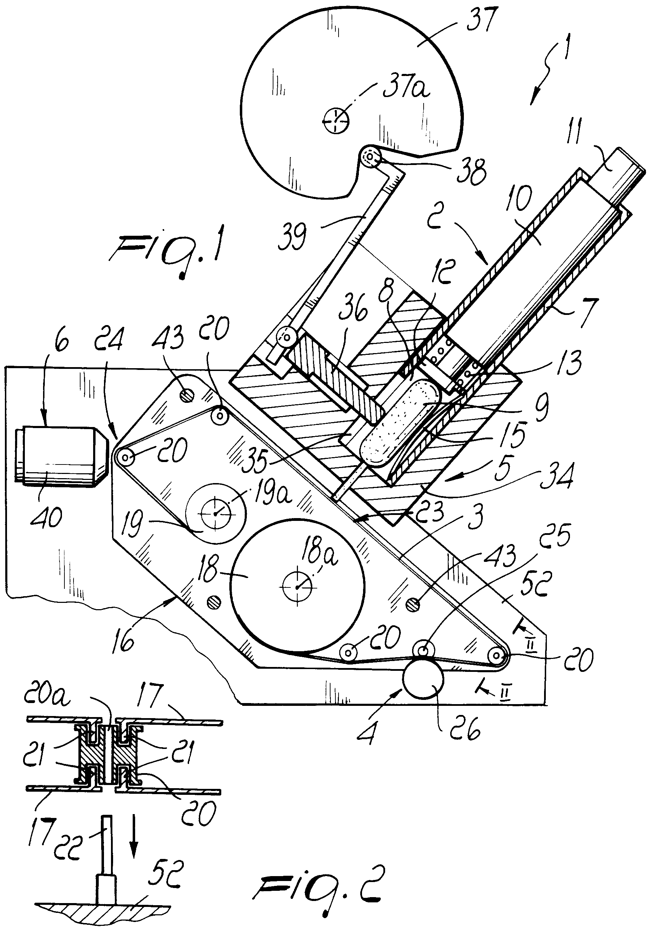

figure 1 is a schematic view of the apparatus for carrying out the method according to the invention;
figure 2 is an enlarged-scale sectional view of a detail of figure 1, taken along the plane II-II of figure 1;
figure 3 is a schematic partially sectional lateral elevation view of the means for drawing a sample of the liquid to be analyzed during sample-taking;
figure 4 is a view, similar to figure 3, of the sampling means after taking a sample of the liquid to be analyzed;

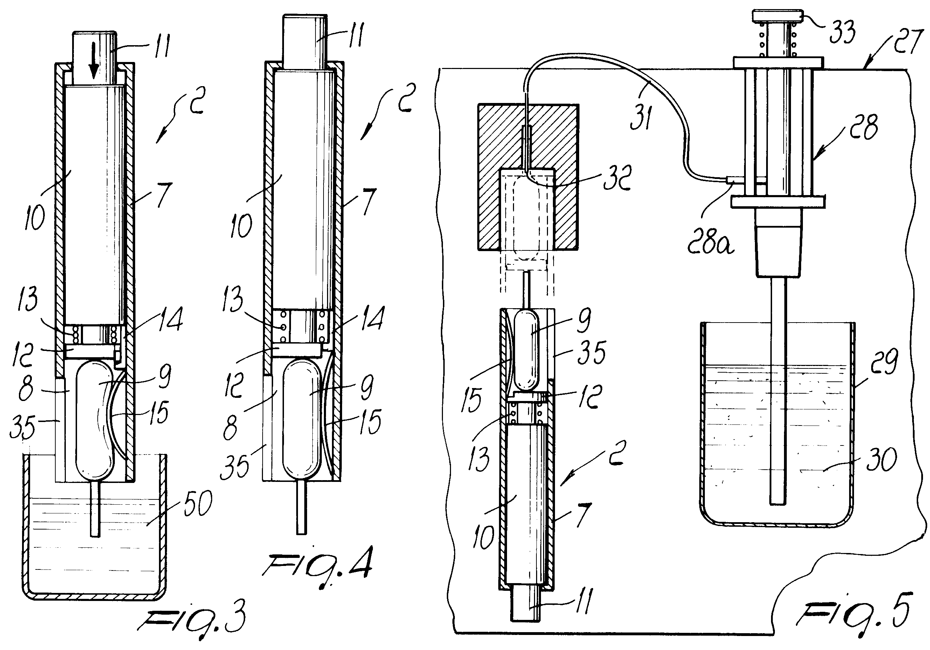

figure 5 is a schematic view of a device for marking the sample of liquid to be analyzed with fluorescent liquid.

With reference to the above figures, the apparatus for carrying out the method according to the invention is generally designated by the reference numeral 1 and comprises: means 2 for drawing a sample of the liquid to be analyzed; a ribbon-like element 3, which runs along a preset path; means 4 for moving the ribbon-like element 3 along its path; means 5 for delivering a film of the sample liquid onto one face of the ribbon-like element 3; and means 6 for the optical analysis of the film of the sample liquid spread on the ribbon-like element 3.

The sampling means 2 comprise a pipette holder 7 that is substantially constituted by a hollow cylindrical body having, at one of its longitudinal ends, a seat 8 inside which a disposable pipette 9 is arranged; said pipette is used to draw a preset amount of the liquid to be analyzed. A piston-like element 10 is accommodated inside the hollow cylindrical body of the pipette holder 7 and protrudes, with a pushbutton-shaped portion 11, from the axial end lying opposite to the seat 8. A fixed abutment 12 is provided inside the pipette holder 7, and a spring 13 is arranged between said fixed abutment 12 and the longitudinal end of the piston-like element 10 lying opposite to the pushbutton-shaped portion 11; said spring 13 elastically biases the advancement of the piston-like element 10 towards the seat 8. The piston-like element 10 is provided, at the end directed towards the seat 8, with an extension 14 that passes through the fixed abutment 12 and acts on a leaf spring 15 which is applied to a side wall of the seat 8 and abuts against the pipette 9 which is inserted in said seat 8.

In practice, by pressing the pushbutton 11, the piston-like element 10 is moved so that its acts, with the extension 14, on the leaf spring 15, making it flex. This flexing of the leaf spring 15 causes the compression of the pipette 9. The release of the pushbutton 11 causes the piston-like element 10 to return to its initial position by virtue of the spring 13 and unloads the leaf spring 15, which by returning to the inactive position allows the pipette 9 to resume its inactive condition, so as to cause a preset amount of liquid 50 to be analyzed to be drawn into the pipette 9.

The ribbon-like element 3 is supported by a replaceable cartridge 16 which is detachably supported inside a compartment formed by the supporting structure 52 of the device.

More particularly, the cartridge 16 is composed of a casing 17 that internally supports a reel 18 of the ribbon-like element 3 so that it can rotate about its axis 18a. A takeup drum 19 for the ribbon-like element is also provided inside the casing 17 and is arranged so that its axis 19a is parallel to the axis 18a of the reel 18. The ribbon-like element 3 unwinds from the reel 18 to the takeup drum 19 along a path defined by rollers 20 which are supported by the casing 17 so that they can rotate freely about their respective axes, which are arranged parallel to the axes 19a and 18a. As shown particularly by figure 2, the casing 17 is internally provided with protrusions 21 that support the rollers 20 so that they can rotate freely about their respective axes. Said rollers 20 are crossed by an axial hole 20a that can be freely engaged by shafts 22 supported by the supporting structure 52 of the apparatus inside the seat meant to contain the replaceable cartridge 16, so as to allow to correctly position the cartridge 16 inside the supporting structure 52 of the apparatus. The cartridge 16 is furthermore fixed in manner known per se inside the seat of the supporting structure 52, for example by means of removable screws 43, knurls, or the like.

The casing 17 of the cartridge is provided, along the extension of the ribbon-like element 3, with at least one first opening 23 located at the delivery means 5 and with at least one second opening 24 arranged at the analysis means 6.

The means 4 for moving the ribbon-like element 3 along the path formed by the rollers 20 include a capstan 25 with a corresponding presser wheel 26 that engages the ribbon-like element 3. The capstan 25 is rotated about its axis by means of an electric motor, not shown for the sake of simplicity, which is also connected, by means of an appropriate clutch system, to the takeup drum 19 so as to gradually unwind the ribbon-like element 3 from the reel 18 and take it up on the drum 19, causing the ribbon-like element 3 to move along the path formed by the rollers 20.

The ribbon-like element 3 is preferably constituted by a ribbon made of non-fluorescent synthetic material.

The apparatus furthermore comprises means 27 for the dosage of a fluorescent marker liquid inside the pipette 9. Said dosage means are constituted by a volumetric pump 28 that draws from a container 29 for the fluorescent marker liquid 30 and is connected, by means of its delivery duct 28a, to a duct 31 that feeds a delivery nozzle 32, at which the inlet of the pipette 9 is placed by using the pipette holder 7 itself.

The volumetric pump 28, which can be operated manually by means of a pushbutton 33 located on the supporting structure 52, delivers a metered amount of the marker liquid 30 inside the pipette 9.

The delivery means 5 comprise a support 34 for the pipette holder 7 which is conveniently provided, proximate to the seat 8, with a lateral opening 35 that is located at a presser element 36 by arranging the pipette holder 7 on the support 34. The presser element 36 is actuated by a cam 37 that is controllably actuated so as to rotate about a rotation axis 37a. The profile of the cam 37 acts on a cam follower 38 which is connected to the presser element 36 by means of a lever 39 so as to move the presser element 36 along a direction that lies transversely to the axis of the pipette 9 in order to gradually compress it, thus delivering a preset amount of the liquid contained inside said pipette. When the pipette holder 7 is arranged on the support 34, the mouth of the pipette 9 faces the opening 23, and at said opening the path of the ribbon-like element 3 conveniently runs along a plane that is inclined with respect to an ideal horizontal plane so that, when the pipette 9 is compressed, an even film of liquid is delivered onto the face of the ribbon-like element 3 that is directed towards the outside of the cartridge 16.

The optical analysis means comprise a known type of fluorescence microscope 40, which faces the second opening 24, arranged downstream of the opening 23 along the direction of the translatory motion of the ribbon-like element 3 along its path.

Use of the apparatus in carrying out the method according to the invention is as follows.

By means of the disposable pipette 9 located in the pipette holder 7, the operator draws a preset amount of the liquid to be analyzed and then places the pipette 9 so that its mouth is located at the nozzle 32, injecting into the pipette a preset amount of the marker liquid 30 by operating the pump 28. The disposable pipette 9 is then arranged, by means of the pipette holder 7, on the support 34 so that the mouth of the pipette faces the ribbon-like element 3 at the opening 23.

The subsequent actuation of the cam 37 causes the sample liquid thus marked to be delivered onto the ribbon-like element 3 during the movement of said element from the reel 18 onto the takeup drum 19. By virtue of the inclination of the ribbon-like element 3 at the opening 23, one achieves an even distribution of the liquid along the ribbon-like element 3 which, during its travel from the reel 18 to the takeup drum 19, causes the liquid to pass in front of the microscope 40, which analyzes said liquid.

After analysis, the pipette 9 can be replaced with a new pipette to perform a new analysis until the reel 18 is depleted. Once the reel 18 has been depleted, the cartridge 16 is replaced with a new one provided with a new ribbon-like element 3 to carry out new analyses.

Owing to the fact that the pipette 9 is not handled directly by the operator but is always handled with the aid of the pipette holder 7, and to the fact that the pipette 9 is replaced for each analysis, the possibility of contamination of the sample of liquid is assuredly eliminated.

Furthermore, by virtue of the fact that the ribbon-like element 3 is unwound from the reel 18 and wound onto the drum 19 during the execution of the analyses, one achieves a further assurance against the risk of contamination of the sample without requiring any washing of the apparatus.

In practice it has been observed that the method according to the invention and the apparatus for carrying it out fully achieve the intended aim, since they allow to analyze the milk directly in the stable, with considerably lower costs than entailed by conventional types of apparatus and of analysis method.

Although the method and the apparatus for carrying it out have been conceived in particular for counting somatic cells in vaccine milk, they can nonetheless be used advantageously also to carry out the analysis of other heterogeneous liquids.

The method and apparatus thus conceived are susceptible of numerous modifications and variations, all of which are within the scope of the inventive concept; all the details may furthermore be replaced with other technically equivalent elements.

In practice, the materials employed, as well as the dimensions, may be any according to the requirements and according to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Method for analyzing heterogeneous liquids, particularly for counting somatic cells in the vaccine milk, characterized in that it consists in taking a sample of the liquid to be analyzed; in evenly spreading a film of the sample liquid on a ribbon-like element that can move along a preset path; in analyzing the film of liquid placed on said ribbon-like element by means of a microscope arranged along said path downstream of the station for spreading said liquid onto the ribbon-like element along the direction of motion of said ribbon-like element.

2. Method according to claim 1, characterized in that said ribbon-like element is made of a non-fluorescent material; in that said sample liquid is marked with fluorescent substances before arriving at said microscope; and in that said microscope is constituted by a fluorescence microscope.

3. Method according to claim 1, characterized in that said sample of liquid is taken by means of a disposable pipette.

4. Method according to one or more of the preceding claims, characterized in that said ribbon-like element is disposable.

5. Apparatus for analyzing heterogeneous liquids, particularly for counting somatic cells in vaccine milk, characterized in that it comprises: means for taking a sample of the liquid to be analyzed; a ribbon-like element that runs along a preset path; means for moving said ribbon-like element along said path; means for delivering a film of the liquid of said sample onto one face of said ribbon-like element, said delivery means facing said ribbon-like element; and means for the optical analysis of the film of sample liquid spread onto said ribbon-like element, said means being arranged downstream of said delivery means along the direction of motion of said ribbon-like element along said path.

6. Apparatus according to claim 5, characterized in that said ribbon-like element is supported by a replaceable cartridge that is detachably supported inside a compartment of a supporting structure of the apparatus.

7. Apparatus according to claims 5 and 6, characterized in that said cartridge comprises a casing that internally supports a reel of said ribbon-like element and a takeup drum for said ribbon-like element, said ribbon-like element unwinding from said reel to said takeup drum along a path defined by rollers whose axes are parallel to the axes of said reel and of said takeup drum, said rollers being freely supported by said casing about their respective axes, said casing having at least one first opening to allow access to said ribbon-like element that faces said delivery means and at least one second opening that faces said analysis means.

8. Apparatus according to one or more of the preceding claims, characterized in that said means for the movement of said ribbon-like element comprise a capstan that engages said ribbon-like element along said path and motor means that are connected to said capstan and to said takeup drum for their actuation.

9. Apparatus according to one or more of the preceding claims, characterized in that said sampling means comprise a pipette holder which is provided with a seat for a disposable sampling pipette and with means for compressing said pipette, to allow said pipette to draw a preset amount of liquid to be analyzed.

10. Apparatus according to one or more of the preceding claims, characterized in that it comprises means for metering a fluorescent marker liquid into said pipette.

11. Apparatus according to one or more of the preceding claims, characterized in that said delivery means comprise a support for said pipette holder, which is arranged at said first opening of the casing of said cartridge, and the presser means acting on said pipette to deliver a film of liquid, of the drawn sample, onto a face of said ribbon-like element.

12. Apparatus according to one or more of the preceding claims, characterized in that said optical analysis means comprise a fluorescence microscope that faces said second opening of the casing of said cartridge.

13. Apparatus according to one or more of the preceding claims, characterized in that said ribbon-like element extends, at said delivery means, along a plane that is inclined with respect to an ideal horizontal plane.

14. Apparatus according to one or more of the preceding claims, characterized in that said supporting structure has, inside said seat, shafts that can be freely coupled to a seat formed coaxially in said rollers.

15. Apparatus according to one or more of the preceding claims, characterized in that said ribbon-like element is made of non-fluorescent synthetic material.
